(19) **Europäisches Patentamt / European Patent Office / Office européen des brevets**

(11) **EP 3 203 402 B1**

(12) **EUROPEAN PATENT SPECIFICATION**

(45) Date of publication and mention
of the grant of the patent:
**28.07.2021 Bulletin 2021/30**

(51) Int Cl.:
**G16C 20/30** *(2019.01)*      **G16C 10/00** *(2019.01)*
**G16B 15/00** *(2019.01)*      G16C 20/50 *(2019.01)*

(21) Application number: **14903465.4**

(22) Date of filing: **03.10.2014**

(86) International application number:
**PCT/JP2014/076549**

(87) International publication number:
**WO 2016/051587 (07.04.2016 Gazette 2016/14)**

(54) **METHOD FOR CALCULATING INTERACTION ENERGY, CALCULATION DEVICE, PROGRAM, AND RECORDING MEDIUM**

VERFAHREN ZUR BERECHNUNG VON WECHSELWIRKUNGSENERGIE, BERECHNUNGSVORRICHTUNG, PROGRAMM UND AUFZEICHNUNGSMEDIUM

PROCÉDÉ POUR CALCULER UNE ÉNERGIE D'INTERACTION, DISPOSITIF DE CALCUL, PROGRAMME, ET SUPPORT D'ENREGISTREMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**09.08.2017 Bulletin 2017/32**

(73) Proprietor: **Fujitsu Limited**
**Kawasaki-shi, Kanagawa 211-8588 (JP)**

(72) Inventor: **SATO, Hiroyuki**
**Kawasaki-shi**
**Kanagawa 211-8588 (JP)**

(74) Representative: **Haseltine Lake Kempner LLP**
**Cheapside House**
**138 Cheapside**
**London**
**EC2V 6BJ (GB)**

(56) References cited:
**JP-A- H09 147 139     JP-A- 2007 511 470**
**JP-A- 2012 083 966**

• HOU T. ET AL: "Predictions of Binding of a Diverse Set of Ligands to Gelatinase-A by a Combination of Molecular Dynamics and Continuum Solvent Models", JOURNAL OF PHYSICAL CHEMISTRY PART B: CONDENSED MATTER, MATERIALS, SURFACES, INTERFACES & BIOPHYSICAL, vol. 106, no. 21, 1 May 2002 (2002-05-01), pages 5527-5535, XP055463347, US ISSN: 1520-6106, DOI: 10.1021/jp015516z

• PAN D. ET AL: "Exploring the molecular basis of dsRNA recognition by NS1 protein of influenza A virus using molecular dynamics simulation and free energy calculation", ANTIVIRAL RESEARCH, ELSEVIER BV, NL, vol. 92, no. 3, 23 September 2011 (2011-09-23), pages 424-433, XP028113949, ISSN: 0166-3542, DOI: 10.1016/J.ANTIVIRAL.2011.09.009 [retrieved on 2011-10-04]

• SEEBER M. ET AL: "Wordom: A user-friendly program for the analysis of molecular structures, trajectories, and free energy surfaces", JOURNAL OF COMPUTATIONAL CHEMISTRY., vol. 32, no. 6, 29 November 2010 (2010-11-29), pages 1183-1194, XP055463344, GB ISSN: 0192-8651, DOI: 10.1002/jcc.21688

• SEEBER M. ET AL: "Wordom: a program for efficient analysis of molecular dynamics simulations", BIOINFORMATICS., vol. 23, no. 19, 23 August 2007 (2007-08-23), pages 2625-2627, XP055463341, GB ISSN: 1367-4803, DOI: 10.1093/bioinformatics/btm378

- **SHAO J. ET AL: "Clustering Molecular Dynamics Trajectories: 1. Characterizing the Performance of Different Clustering Algorithms", JOURNAL OF CHEMICAL THEORY AND COMPUTATION, vol. 3, no. 6, 6 October 2007 (2007-10-06) , pages 2312-2334, XP009504451, DOI: 10.1021/ct700119m**
- **ROE D. R. ET AL: "PTRAJ and CPPTRAJ: Software for Processing and Analysis of Molecular Dynamics Trajectory Data", JOURNAL OF CHEMICAL THEORY AND COMPUTATION, vol. 9, no. 7, 10 June 2013 (2013-06-10), pages 3084-3095, XP009504452, American Chemical Society, Washington, DC DOI: 10.1021/ct400341p**
- **BREHM M. ET AL: "TRAVIS - A Free Analyzer and Visualizer for Monte Carlo and Molecular Dynamics Trajectories", JOURNAL OF CHEMICAL INFORMATION AND MODELING, vol. 51, no. 8, 16 July 2011 (2011-07-16), pages 2007-2023, XP009504403, American Chemical Society, Washington, DC DOI: 10.1021/ci200217w**
- **YUSUKE MATSUZAKI ET AL.: 'Protein structure sampling based on molecular dynamics and improvement of docking prediction' IPSJ SIG NOTES HEISEI 21 NENDO vol. 3, no. 18, 15 October 2009, pages 1 - 6, XP008184385**
- **SHIN'ICHI TOKUNAGA ET AL.: 'Analysis of protein folding using probabilistic information processing' IPSJ SIG NOTES vol. 2007, no. 128, 20 December 2007, pages 275 - 280, XP008184384**
- **YASUHIRO IIHASHI AMBER KENKYUKAI, AMBER NI YORU SEITAI KOBUNSHI SIMULATION NYUMON 02 June 2011, pages 105 - 115, XP008185604**
- **AKIFUMI KATO ET AL.: 'BIO IMAGING BIOSTATION VIEWER: ANALYSIS AND VISUALIZATION FOR INTERACTIONS OF BIO-MACROMOLECULES' JOURNAL OF THE VISUALIZATION SOCIETY OF JAPAN vol. 26, no. 101, 01 April 2006, pages 2 - 7, XP055362365**

**Description**

The embodiments discussed herein relate to a method for calculating interaction energy between target molecules and drug candidate molecules and a calculation device, a program for causing a computer to execute the calculation method, and a recording medium comprising the program stored thereon.

In recent years, various simulations have been performed by calculators in order to reduce enormous costs and efforts spent on experimentally calculating drug candidate molecules. The calculation of a drug candidate molecule is to calculate a molecule (ligand) strongly interacting with a target molecule associated with a target disease (a targeted disease) as a drug candidate. Screening of a compound based on a target molecular steric structure by means of calculators has been actively performed.

As a judgement whether a bond between protein that is a target molecule and a ligand that is a drug candidate molecule is stable, for example, energy associated with interaction can be highly accurately determined by a method based on quantum mechanics (QM) (see, for example, NPL1). A structure of a complex of protein and a ligand often used in the determination is a crystalline structure obtained by an experiment. There is however a problem that the crystalline structure is different from a structure at room temperature and being covered with water molecules in vivo.

When it is desired to obtain a structure of a complex of protein and a ligand in vivo, use of a biological simulation according to molecular dynamics (MD) is considered. In the MD simulation, however, a structure defers per time period because a force and speed applied on each atom are calculated on each time period, and a position of each atom is determined with a sequential time. Accordingly, actual interaction energy cannot be appropriately determined by calculating interaction energy with a structure of the complex for one time period. In order to determine appropriate interaction energy, it is necessary to calculate interaction energy of structures of the complex for a plurality of time periods, and perform statistical processing.

When interaction energy of structures of a complex for a plurality of time periods is to be calculated, it is necessary to select many structures in order to obtain a statistically excellent result. However, to calculate all of the selected structures by a method based on quantum mechanics takes a long time for calculation, hence such a calculation is difficult.

Citation List

Non-Patent Literature

NPL 1: The Fragment Molecular Orbital Method: Practical Applications to Large Molecular Systems., CRC Press (2009) ISBN/ISSN:1420078488 NPL 2: Hou T. et al. (2002) "Predictions of Binding of a Diverse Set of Ligands to Gelatinase-A by a Combination of Molecular Dynamics and Continuum Solvent Models", JOURNAL OF PHYSICAL CHEMISTRY PART B, vol. 106, no. 21, pages 5527-553

NPL 3: Pan D. et al. (2011) "Exploring the molecular basis of dsRNA recognition by NS1 protein of influenza A virus using molecular dynamics simulation and free energy calculation", ANTIVIRAL RESEARCH, vol. 92, no. 3, pages 424-433

It is desirable to provide a method for calculating interaction energy and a calculation device, both of which can efficiently calculate appropriate interaction energy between target molecules and drug candidate molecules, a program for executing the calculation method, and a recording medium comprising the program. Aspects of embodiments of the disclosure provide a method for calculating interaction energy between a target molecule and a drug candidate, a program for causing a computer to execute the method, a computer-readable medium comprising the program, and a calculating device comprising the computer-readable medium, all as defined in the claims.

The scope of the invention is defined by the claims.

The disclosed method for calculating interaction energy may solve the above-described various problems existing in the art, achieve the above-described desirable outcomes, and efficiently calculate appropriate interaction energy between a target molecule and a drug candidate molecule.

The disclosed program may solve the above-described various problems existing in the art, achieve the above-described desirable outcomes, and efficiently calculate appropriate interaction energy between a target molecule and a drug candidate molecule.

The disclosed computer-readable recording medium may solve the above-described various problems existing in the art, achieve the above-described desirable outcomes, and efficiently calculate appropriate interaction energy between a target molecule and a drug candidate molecule.

The disclosed calculation device may solve the above-described various problems existing in the art, achieve the above-described desirable outcomes, and efficiently calculate appropriate interaction energy between a target molecule and a drug candidate molecule.

The invention is described with reference to the Figures, in which:

FIG. 1 is a graph depicting one example of groups of resultant structures of the MD simulation using RMSD.
FIG. 2A is a schematic view for explaining a step including dividing groups (part 1).
FIG. 2B is a schematic view for explaining a step including dividing groups (part 2).
FIG. 2C is a schematic view for explaining a step including dividing groups (part 3).
FIG. 2D is a graph for explaining a step including dividing groups.
FIG. 3 is a flow-chart depicting one example of the disclosed method for calculating interaction energy.
FIG. 4 is a hardware configuration example of the disclosed device.
FIG. 5 is a data configuration example of a memory unit.
FIG. 6 is a graph depicting a result of grouping of Example 1.
FIG. 7 is a graph depicting a result of grouping of Comparative Example 1.

Description of Embodiments

(Method for calculating interaction energy)

The disclosed method for calculating interaction energy is a method for calculating interaction energy between target molecules and drug candidate molecules using a calculator.

The method for calculating interaction energy comprises at least a step including dividing into groups, preferably further includes a step including calculating an expected value (E) of interaction energy, and may further include other steps according to the necessity.

The method for calculating interaction energy is performed using a calculator. The number of the calculators used for performing the method for calculating interaction energy may be 1, or 2 or more. For example, a plurality of the calculator may be allowed to execute the method for calculating interaction energy with decentralizing.

In the step including dividing into groups, dividing a trajectory over a total time duration of a molecular dynamic simulation of the target molecule and the drug candidate molecule into groups based on molecular mechanic interaction energy between the target molecule and the drug candidate molecule calculated by molecular mechanics.

Note that, in the scope of claims and the present specification, interaction energy calculated by molecular mechanics may be referred to as "molecular mechanic interaction energy" in order to clarify a difference with interaction energy in an electron state based on quantum mechanics.

A typical method for dividing a trajectory of a molecular dynamic (MD) simulation into groups is a method using root-mean-square deviation (RMSD) that is an index representing deviation of resultant structures.

A value of RMSD of Structure B relative to Structure A can be represented by the following mathematical formula.

[Mathematical Formula 1]

$$RMSD(B) = \sqrt{\frac{1}{N}\sum_{i=1}^{N}(b_i - a_i)^2}$$

In the formula above, $a_i$ is an "i"th coordinate of Structure A where i is a number, $b_i$ is an "i"th coordinate of Structure B, and N is the total number of coordinates.

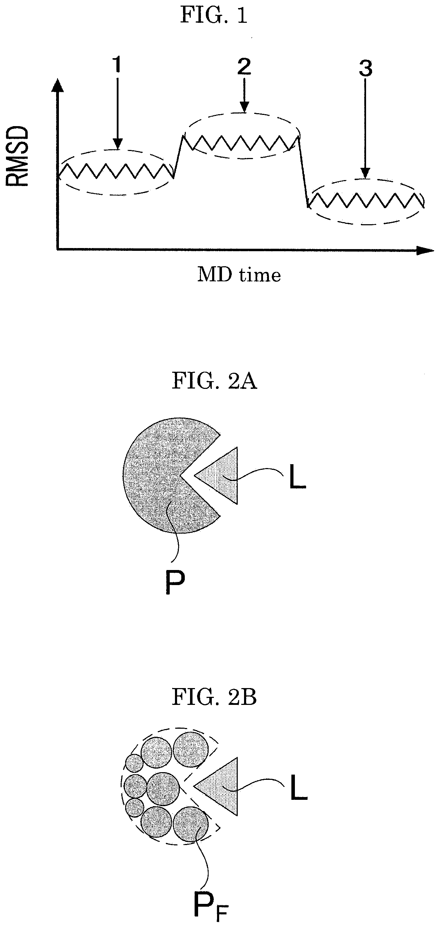

FIG. 1 illustrates one example of grouping of a trajectory of a MD simulation using RMSD. As illustrated in FIG. 1, RMSD in the MD simulation is calculated to determine a relationship between the MD time period and RMSD. The resultant structures are divided into groups 1, 2, and 3.

In case of a large scale structure, such as protein and a ligand, however, the slightest difference of RMSD leads to a large difference in interaction energy. Therefore, it is difficult to divide into appropriate groups according to the above-described method.

Accordingly, the present inventors have found that a trajectory of a MD simulation is divided into groups based on interaction energy of molecular mechanics (MM), not based on an index related to the structure (e.g., RMSD). As a result of grouping based on the molecular mechanic interaction energy, it is possible to classify complexes having similar interaction into the same group among complexes of a target molecule and a drug candidate molecule obtained in each time period of the MD simulation. The interaction energy based on MM may have an absolute value that is less accurate compared to interaction energy based on quantum mechanics, but accuracy of a relative value of the MM interaction energy is high. Therefore, a problem in accuracy can be solved by focusing on only a deviation of the MM interaction energy.

The target molecule is not particularly limited and may be appropriately selected depending on the intended purpose. Examples of the target molecule include protein, ribonucleic acid (RNA), and deoxyribonucleic acid (DNA).

<Grouping>

In the grouping, a trajectory over a total time duration of the molecular dynamic simulation of the target molecule and the drug candidate molecule is divided into groups based on the molecular mechanic interaction energy of the target molecule and the drug candidate molecule calculated by molecular mechanics.

In the molecular mechanics (MM) (molecular force field calculation), interaction energy (the molecular mechanic interaction energy) is calculated using classical mechanics. When the interaction energy is calculated, a molecular force field is used. The molecular force field is a function for applying potential energy of a molecule.

The molecular force field used when the interaction energy is calculated by the molecular mechanics is not particularly limited and may be appropriately selected depending on the intended purpose. Examples of the molecular force field include the AMBER molecular force field, the CHARMm molecular force field, and the GROMOS molecular force field. For example, the interaction energy is calculated using a non-bonded part of any of the above-listed molecular force fields. Examples of the nonbonding item include electrostatic energy and van der Waals energy.

The molecular dynamic calculation with which the molecular dynamic simulation is performed is based on classical mechanics.

The molecular dynamic simulation can be performed using a molecular dynamic calculation program. Examples of the molecular dynamic calculation program include AMBER, CHARMm, GROMACS, GROMOS, NAMD, and myPresto.

The trajectory means a time-series structural change in the molecular dynamic simulation.

In the grouping, the following three processes [a process for obtaining an average value ($E_M$), a process for extracting fragments, and a process for dividing into groups] are preferably performed in this order.

The computational complexity can be further reduced without losing accuracy of the calculation by dividing the target molecule into the fragments, and performing the above-mentioned three processes.

<<Process for obtaining an average value ($E_M$)>>

The process for obtaining an average value ($E_M$) is a process including obtaining an average value ($E_M$) of molecular mechanic interaction energy of each of fragments and the drug candidate molecules per a predetermined unit time within the total time duration, for each of the fragments over the total time duration, where the fragments are obtained by dividing the target molecule into partial structures, and the molecular mechanic interaction energy is calculated over the total time duration of the molecular dynamic simulation of the target molecule and the drug candidate molecule.

The molecular mechanic interaction energy in the process for obtaining an average value ($E_M$) can be calculated by molecular mechanics.

The molecular mechanic interaction energy is interaction energy calculated over a total time duration of the molecular dynamic simulation of the target molecule and the drug candidate molecule.

The molecular mechanic interaction energy is interaction energy between each of fragments and the drug candidate molecule, where the fragments are obtained by dividing the target molecule into partial structures.

The fragments are obtained by dividing the target molecule into partial structures. A method for dividing the target molecule into the partial structures is not particularly limited and may be appropriately selected depending on the intended purpose. Examples of the method include a method where the target molecule is divided into partial structures by amino acid residue units.

The division of the target molecule into the fragments may be performed by an automatic setting of a calculator, or by manually inputting various conditions to a calculator.

The average value ($E_M$) of the molecular mechanic interaction energy is not particularly limited and may be appropriately selected depending on the intended purpose. Examples of the average value ($E_M$) include a moving average value. Examples of moving average for determining the moving average value include simple moving average, weighted moving average, and exponential moving average.

The total time duration of the molecular dynamic simulation is not particularly limited and may be appropriately selected depending on the intended purpose. For example, the total time duration of the molecular dynamic simulation is from 100 ns to 1,000 ns.

The predetermined unit time is not particularly limited and may be appropriately selected depending on the intended purpose. For example, the predetermined unit time is from 1 ps to 100 ps. The predetermined time unit may be identical to or different from an interval (time step) between one snapshot and a subsequent snapshot of the molecular dynamic simulation. For example, the predetermined unit time may be a time interval between one snapshot and a snapshot coming a few snapshots after the one snapshot.

The average values ($E_M$) are obtained for each fragment.

The average values ($E_M$) are obtained over the total time duration.

The number of the average values (EM) for the fragments is a number obtained by dividing the total time duration by the predetermined unit time.

<<Process for extracting fragments>>

The process for extracting fragments is a process including extracting fragments having large interaction with the drug candidate molecule from all of the fragments using the average values $(E_M)$ and a predetermined threshold A.

In the process for extracting fragments, fragments having large interaction with the drug candidate molecule can be extracted by selecting fragments using the threshold A. The degree of the interaction is not particularly limited and may be appropriately selected depending on the intended purpose.

The number of the threshold A set when the process for extracting fragments is performed is typically one.

The threshold A is not particularly limited and may be appropriately selected depending on the intended purpose. For example, the threshold A is appropriately set depending on the average value $(E_M)$ obtained for each of the fragments.

The threshold A may be automatically set by a calculator, or may be input manually into the calculator.

Examples of the process for extracting fragments include a process where a representative average value $(E_{RM})$ representing all of the average values $(E_M)$ of each of the fragments is determined for each of the fragments, and the fragments are extracted when the representative average value $(E_{RM})$ is the threshold A or greater.

Examples of the representative average value $(E_{RM})$ include a maximum value of all of the average values $(E_M)$, and an arithmetic mean value of all of the average values $(E_M)$.

<<Process for dividing into groups>>

The process for dividing into groups is a process including dividing the trajectory over the total time duration of the molecular dynamic simulation of the target molecule and the drug candidate molecule into groups using a fluctuation range of the average value $(E_M)$ of each of the fragments extracted during the molecular dynamic simulation, and a predetermined threshold B.

The number of the threshold B set when the process for dividing into groups is performed is typically one.

The threshold B is not particularly limited and may be appropriately selected depending on the intended purpose. For example, the threshold B is appropriately set depending on the fluctuation range.

The threshold B may be automatically set by a calculator, or may be manually input into the calculator.

The fluctuation range means a difference in a value between an average value $(E_M)i$, and an average value $(E_M)i+1$ calculated for the time unit $i+1$ after the time unit $i$ for which the average value $(E_M)i$ is calculated.

Examples of the process for dividing into groups include grouping where the fluctuation range of the average values $(E_M)$ of the extracted fragments is compared to the predetermined threshold B, and a time range when the fluctuation range of each of the fragments is the predetermined threshold B or less is grouped as one group.

One example of the grouping is explained using FIGs. 2A to 2D.

-Determining average value $(E_M)$-

First, protein P is divided into partial structures to obtain a plurality of fragments $P_F$ (FIG. 2B). For example, the fragment PF is one amino acid residue.

Next, molecular mechanic interaction energy between the fragment $P_F$ and the drug candidate molecule L, which is interaction energy calculated over a total time duration of the molecular dynamic simulation, and is calculated by molecular mechanics, is determined. An average value $(E_M)$ of the interaction energy per unit time within the total time duration is determined for each fragment $P_F$ over the total time duration.

The predetermined unit time is, for example, identical to time increments of a molecular dynamic simulation.

-Process for extracting fragments-

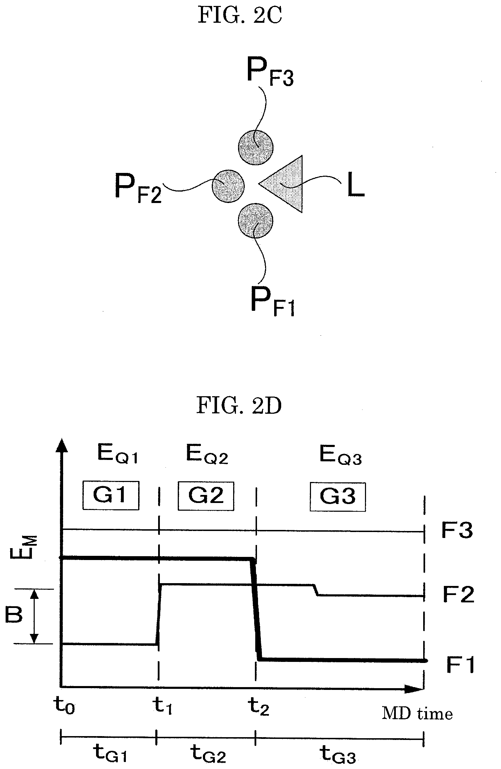

Next, the fragments having large interaction with the drug candidate molecule L are extracted from the fragments $P_F$ using the average values $(E_M)$ and a threshold A. For example, as illustrated in FIG. 2C, fragments $P_{F1}$, $P_{F2}$, and $P_{F3}$ present near the drug candidate molecule L are extracted.

-Process for dividing into groups-

Next, a trajectory over a total time duration of the molecular dynamic simulation of the target molecule P and the drug candidate molecule L is divided into groups using a fluctuation range obtained from the molecular dynamic simulation of the average values $(E_M)$ of the extracted fragments $P_{F1}$, $P_{F2}$, and $P_{F3}$, and the predetermined threshold B.

In FIG. 2D, a fluctuation line F1 of the average value $(E_M)$ of the molecular mechanic interaction energy between the fragment $P_{F1}$ and the drug candidate molecule L, a fluctuation line F2 of the average value $(E_M)$ of the molecular mechanic interaction energy between the fragment $P_{F2}$ and the drug candidate molecule L, and a fluctuation line F3 of the average value ($E_M$) of the molecular mechanic interaction energy between the fragment $P_{F3}$ and the drug candidate molecule L are plotted in a graph. The time ranges when the fluctuation range of each of the fluctuation lines F1, F2, and F3 is the threshold B or less are grouped as groups G1 to G3. For example, the group G1 is a group which includes from the initial stage (to) of the MD simulation to the time ($t_1$). At the time (ti), the fluctuation range of the fluctuation line F2 is greater than the threshold B. Accordingly, the time range from the MD initial stage ($t_0$) to the time ($t_1$) is grouped as one group. Although there is hardly any deviation in the fluctuation lines F1, F2, and F3 from the time ($t_1$) to the time ($t_2$), the fluctuation range of the fluctuation line F1 is large at the time ($t_2$) and is greater than the threshold B. Accordingly, the time range from the time ($t_1$) to the time ($t_2$) is grouped as one group (the group G2). There is a slight deviation in the fluctuation line F2 from the time ($t_2$) to the termination of the MD simulation, but the deviation is not a fluctuation range greater than the threshold B, and there is hardly any deviation in the fluctuation lines F1 and F3. Accordingly, the time range from the time ($t_2$) to the termination of the MD simulation is grouped as one group (the group G3).

<Calculation of expected value (E)>

In the calculation of the expected value (E), a representative structure for the target molecule and the drug candidate molecule is determined in each of the groups, interaction energy ($E_Q$) of an electron state based on quantum mechanics is calculated for the determined representative structure of each of the groups, and an expected value (E) of interaction energy is calculated from the calculated interaction energy ($E_Q$) of the representative structure of each of the groups and a duration of each of the groups.

The representative structure is a structure of a complex of the target molecule and the drug candidate molecule, and a structure representing each of the groups.

A method for determining the representative structure for each of the groups is not particularly limited and may be appropriately selected depending on the intended purpose. Examples of the method include a method where an average value of a coordinate of each of atoms constituting the complex in the group is determined, and the obtained average coordinate is determined as a representative structure, and a method where a resultant structure having the average value of the average value ($E_M$) of the group, or similar to the average value ($E_M$) is determined as a representative structure.

The interaction energy ($E_Q$) is determined by molecular orbital calculation according to a molecular orbital method. Examples of the molecular orbital calculation include nonempirical molecular orbital calculation (ab initio molecular orbital calculation), and semiempirical molecular orbital calculation.

Examples of a methodology of the nonempirical molecular orbital calculation include the Hartree-Fock method, and the electron correlation method.

Examples of a methodology of the semiempirical molecular orbital calculation include CNDO, INDO, AM1, and PM3.

Examples of a program of the nonempirical molecular orbital calculation include Gaussian03, GAMESS, ABINIT-MP, and Protein DF.

Examples of a program of the semiempirical molecular orbital calculation include MOPAC.

Examples of a method for calculating the expected value (E) include a method using the following mathematical formula.

[Mathematical Formula 2]

$$E = \sum_i t_{Gi} E_{Qi} \Big/ \sum_i t_{Gi}$$

In the formula above, $t_{Gi}$ is an "i"th duration ($t_G$) where i is a number, and $E_{Qi}$ is "i"th interaction energy ($E_Q$).

A specific example of the calculation method of the expected value (E) is explained using FIG. 2D.

A duration of the group G1 is $t_{G1}$, and interaction energy of the representative structure of the group G1 is $E_{Q1}$.

A duration of the group G2 is $t_{G2}$, and interaction energy of the representative structure of the group G2 is $E_{Q2}$.

A duration of the group G3 is $t_{G3}$, and interaction energy of the representative structure of the group G3 is $E_{Q3}$.

Accordingly, an expected value is determined from the mathematical formula above as $E = [(t_{G1} \times E_{Q1}) + (t_{G2} \times E_{Q2}) + (t_{G3} \times E_{Q3})]/(t_{G1} + t_{G2} + t_{G3})$.

In the method for calculating interaction energy, the calculation of the expected value (E) is preferably repeated with fluctuating the predetermined threshold B until a fluctuation value of the expected value (E) is converged on a predetermined value. The repetition of the calculation can improve accuracy of the expected value (E).

The value with which the repetition of the calculation is terminated and the convergence is confirmed is not particularly limited and may be appropriately selected depending on the intended purpose. The value may be automatically set by a calculator, or may be manually input into the calculator.

For example, the method for calculating interaction energy can be performed by means of any of general computer systems (e.g., various network servers, work stations, and personal computers) including central processing units (CPUs), random access memories (RAMs), hard disks, can various computer peripherals.

(Program)

The disclosed program is a program for causing a computer to execute the disclosed method for calculating interaction energy.

A preferable embodiment for executing the method for calculating interaction energy is identical to a preferable embodiment of the method for calculating interaction energy.

The program can be created using any of various programing languages known in the art according to a configuration of a computer system for use, a type or version of an operation system for use.

The program may be recorded on a recording medium, such as an integral hard disk, and an external hard disk, or recorded on a recording medium, such as a compact disc read only memory (CD-ROM), a digital versatile disk read only memory (DVD-ROM), a magneto-optical (MO) disk, and a universal serial bus (USB) memory stick (USB flash drive). In the case where the program is recorded on a recording medium, such as a CD-ROM, a DVD-ROM, an MO disk, and an USB memory stick, the program can be used, as required, directly or by installing a hard disk via a recording medium reader equipped in a computer system. Moreover, the program may be recorded in an external memory region (e.g. another computer) accessible from the computer system via an information and communication network, and the program may be used, as required, by directly from the external memory region or installing into a hard disk from the external memory region via the information and communication network.

The program may be divided into predetermined processes and recorded on a plurality of recording mediums.

(Computer-readable recording medium)

The disclosed computer-readable recording medium comprises the disclosed program stored thereon.

The computer-readable recording medium is not particularly limited and may be appropriately selected depending on the intended purpose. Examples of the computer-readable recording medium include integral hard disks, external hard disks, CD-ROMs, DVD-ROMs, MO disks, and USB memory sticks.

The recording medium may be a plurality of recording mediums to which the program is divided into the predetermined processes and recorded.

(Interaction energy calculation device)

The disclosed interaction energy calculation device includes the disclosed computer-readable recording medium.

The calculation device may be a plurality of calculation devices each including a recording medium, where the program is divided into the predetermined processes and recorded on a plurality of the recording mediums included in the calculation devices.

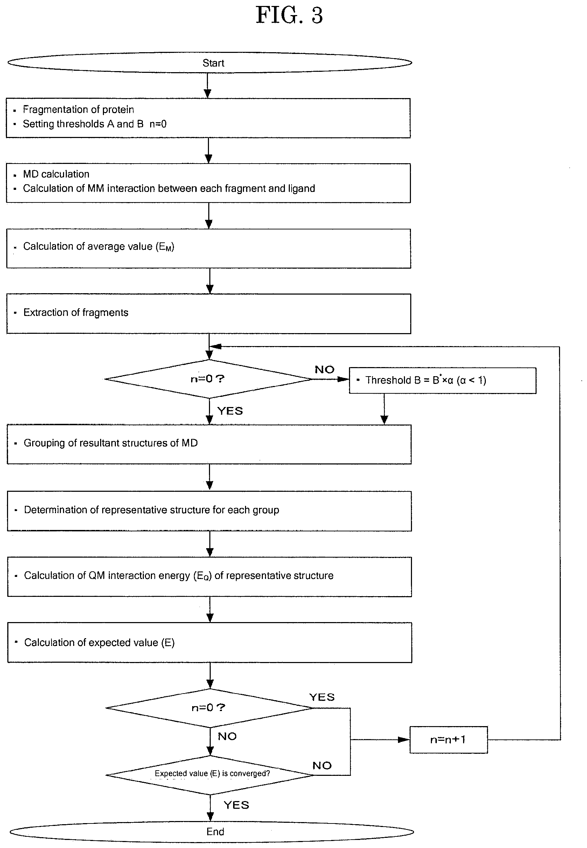

One example of the method for calculating interaction energy is explained with reference to the flow chart of FIG. 3.

In the example as illustrated, protein is used as a target molecule, and a drug candidate molecule is referred to as a ligand.

(1) Fragmentation of protein is performed. Moreover, setting of thresholds A and B is performed. At the time of the setting, n is set to 0 (n=0) as a repeating number.
(2) Next, a molecular dynamic (MD) simulation of the protein and the ligand is performed. Moreover, molecular mechanic (MM) interaction energy between each fragment of the protein and the ligand is calculated.
(3) Next, an average value ($E_M$) per predetermined unit time within the total time duration of the MD simulation is calculated from the calculated MM interaction energy.
(4) Next, extraction of fragments is performed using the average values ($E_M$) and the threshold A.
(5) Next, determination of n is performed. Since a value of n is 0, proceed to the next process without changing the threshold B.
(6) Next, grouping of resultant structures of the MD simulation is performed.

(7) Next, a representative structure for each group is determined.

(8) Next, interaction energy ($E_Q$) based on quantum mechanics (QM) is calculated for each of the determined representative structures.

(9) Next, an expected value (E) is calculated from the interaction energy ($E_Q$) of the representative structure of each group and a duration of each group.

(10) Next, determination of n is performed.

(11) Since a value of n is 0, 1 is added to the repeating number n and the determination of n of (5) is performed.

(12) Since n is 1 in the determination of n, the threshold B is changed. Here, the threshold B is reduced by multiplying the threshold B with a number smaller than 1.

(13) Next, the same processes to the processes (6) to (9) are performed.

(14) Next, determination of n is performed.

(15) Since n is 1, not 0, convergence of the expected value (E) is confirmed. When the convergence of the expected value (E) is confirmed, the calculation is terminated. If the expected value (E) is not converged, on the other hand, 1 is added to the repeating number n, and return to the determination of n of (11) above.

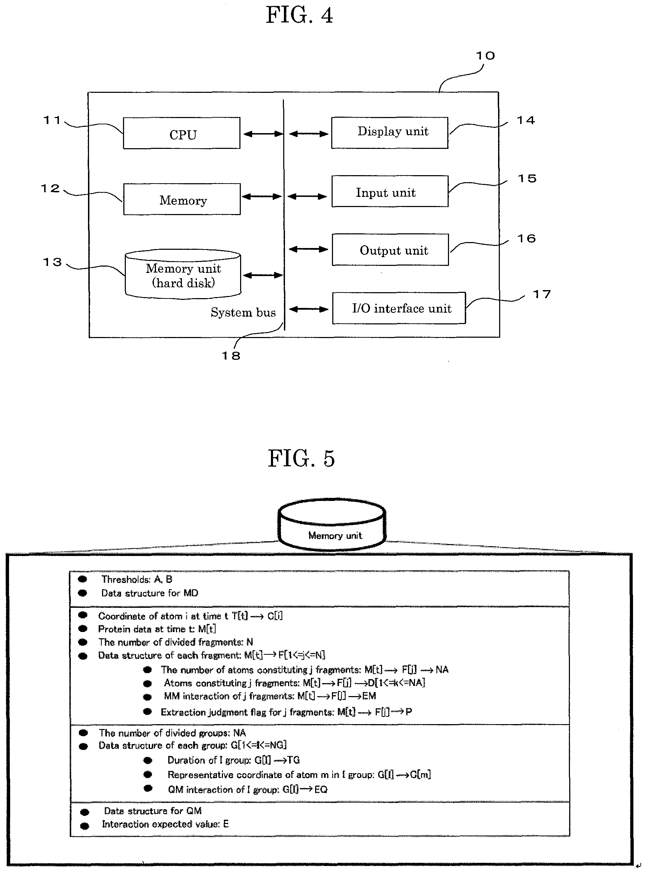

An example of a hardware structure of the disclosed device is illustrated in FIG. 4.

The device 10 is composed by connecting CPU 11, a memory 12, a memory unit 13, a display unit 14, an input unit 15, an output unit 16, and an I/O interface unit 17 via a system bus 18.

The central processing unit (CPU) 11 is configured to perform calculation (e.g., four arithmetic operation, and relational operation), and control of operations of hardware and software.

The memory 12 is a memory, such as a random access memory (RAM), and a read only memory (ROM). The RAM is configured to store an operation system (OS) and application programs read from the ROM and the memory unit 13, and function as a main memory and work area of the CPU 11.

The memory unit 13 is a device for storing various programs and data. For example, the memory unit 13 is a hard disk. In the memory unit 13, programs to be executed by the CPU 11, data required for executing the programs, and an OS are stored.

The program is stored in the memory unit 13, loaded on the RAM (a main memory) of the memory 12, and executed by the CPU 11.

The display unit 14 is a display device. For example, the display unit is a display device, such as a CRT monitor, and a liquid crystal panel.

The input unit 15 is an input device for various types of data. Examples of the input unit include a key board, and a pointing device (e.g., a mouse).

The output unit 16 is an output device for various types of data. For example, the output unit is a printer.

The I/O interface unit 17 is an interface for connecting to various external devices. For example, the I/O interface unit enables input and output of data of CD-ROMs, DVD-ROMs, MO disks, and USB memory sticks.

An example of a data structure of the memory unit is illustrated in FIG. 5.

In one example of the disclosed method for calculating interaction energy, a data structure illustrated in FIG. 5 is generated in the memory unit. For example, the flow illustrated in FIG. 3 is executed using the data structure.

For example, the data structure illustrated in FIG. 5 is generated as follows.

Protein is divided into N pieces of fragments. The number of atoms constituting the fragment j at the time t is set to $M[t] \rightarrow F[j] \rightarrow NA$, the identification number k of the constituting atom is set to $M[t] \rightarrow F[j] \rightarrow D[k]$, and an extraction judgement flag of each fragment is set to $M[t] \rightarrow F[j] \rightarrow P$. The above-described data has separate data for the time t, but the data structure may be a data structure regardless of time.

The MD simulation is performed and a data structure for MD is set. For each time period of MD, MM interaction energy between each fragment of the protein and a ligand is set to $M[t] \rightarrow F[j] \rightarrow EM$.

As for the EM, a moving average deviation during a certain time period F is calculated and updated as EM data. Fragments each having a value of the EM equal to or greater than the threshold A over the total time duration are extracted.

A time period when values of EM of all of the extracted fragments are fluctuated by a value equal to or less than the threshold B is grouped as a group duration. The number of divided groups is set to NG, a duration of I Group is set to $G[I] \rightarrow TG$, and a coordinate of an atom m in the representative structure of I Group is set to $G[I] \rightarrow C[m]$.

QM interaction energy of the representative structure of I Group is calculated and set to $G[I] \rightarrow EQ$.

An expected value (E) of QM interaction energy is obtained by determining a sum of all of values of $[G[I] \rightarrow EQ]*[G[I] \rightarrow TG]$ for I, and dividing the sum by the total time duration of MD.

Examples

The disclosed technique is explained hereinafter.

(Example 1)

Using the disclosed technique, grouping of resultant structures of a molecular dynamic simulation of protein and a ligand was performed.

As the protein and the ligand, 3MY0 of the protein data bank (PDB) was used.

The grouping was performed using interaction energy between fragments of the protein and the ligand.

The fragmentation of the protein was performed using one amino acid residue as a unit.

The threshold A used for extracting fragments was 1/5 or greater the entire resultant structures and was set to 20 kcal/mol.

The MD simulation was performed using GROMACS.

The threshold B used for grouping the trajectory of MD was set to 6 kcal/mol. As a result of the MD simulation and the grouping, the number of the extracted fragments were 3 (Frag1, Frag2, and Frag3). The trajectory of the MD simulation was divided into 15 groups.

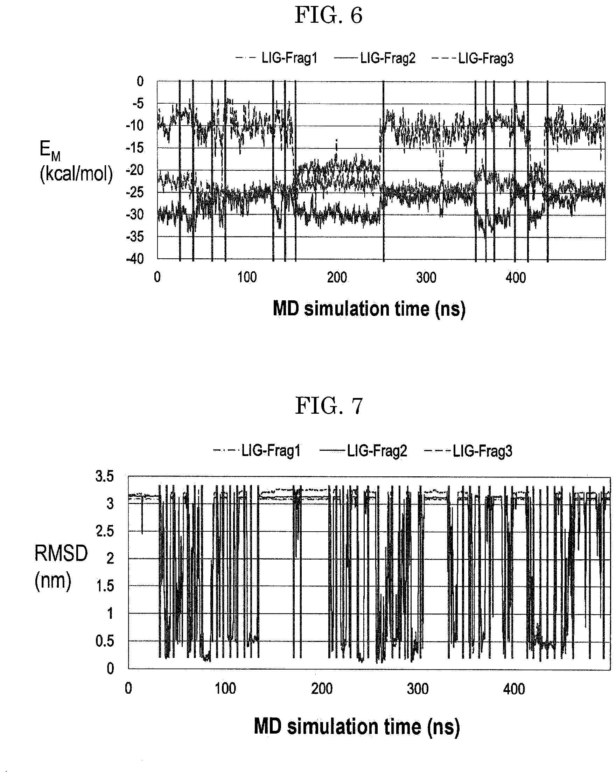

The result is presented in FIG. 6.

(Comparative Example 1)

Grouping of resultant structures of a molecular dynamic simulation of protein and a ligand was performed according to a conventional method.

As the protein and the ligand, 3MY0 of the protein data bank (PDB) was used.

The grouping was performed based on RMSD of a complex of the protein and the ligand in a MD simulation.

The MD simulation was performed in the same manner as in Example 1.

A threshold of RMSD was 2.5 nm, and a level where the RMSD exceeded the threshold was determined as a boundary of a group.

The MD simulation, and the grouping into the three fragment groups identical to Example 1 were performed. As a result, the trajectory of the MD simulation was divided into 43 groups.

The result is presented in FIG. 7.

Comparing Example 1 with Comparative Example 1, the number of groups is smaller in Example 1. Therefore, computational complexity of interaction energy based on quantum mechanics is lower in Example 1. Since grouping was performed based on interaction energy calculated by molecular mechanics in Example 1, moreover, appropriate grouping could be performed, and as a result, reliability of the calculation result of the interaction energy based on mechanics by the MD simulation was enhanced.

Reference Signs List

| | |
|---|---|
| 10 | Device |
| 11 | CPU |
| 12 | Memory |
| 13 | Memory unit |
| 14 | Display unit |
| 15 | Input unit |
| 16 | Output unit |
| 17 | I/O interface unit |
| 18 | System bus |
| L | Drug candidate molecule |
| P | Protein |
| $P_F$ | Fragment |
| $P_{F1}$, $P_{F2}$, $P_{F3}$ | Extracted fragments |

**Claims**

1. A method for calculating interaction energy between a target molecule and a drug candidate molecule using a calculator, the method comprising:
   dividing a trajectory over a total time duration of a molecular dynamic simulation of the target molecule and the drug candidate molecule into groups based on molecular mechanic interaction energy between the target molecule and the drug candidate molecule calculated by molecular mechanics,
wherein the dividing comprises:

obtaining, using the calculator, an average value ($E_M$) of molecular mechanic interaction energy of each of fragments and the drug candidate molecules per a predetermined unit time within the total time duration, for each of the fragments over the total time duration, where the fragments are obtained by dividing the target molecule into partial structures, and the molecular mechanic interaction energy is calculated over the total time duration of the molecular dynamic simulation of the target molecule and the drug candidate molecule;

extracting, using the calculator, the fragments having a large interaction with the drug candidate molecule from all of the fragments using the average values ($E_M$) and a predetermined threshold A, wherein the fragments having the large interaction with the drug candidate molecule are identified as fragments having a representative average value ($E_{RM}$) representing all of the average values ($E_M$) that is equal to or greater than predetermined threshold A; and

dividing, using the calculator, the trajectory over the total time duration of the molecular dynamic simulation of the target molecule and the drug candidate molecule into groups using a fluctuation range of the average value ($E_M$) of each of the fragments extracted during the molecular dynamic simulation, and a predetermined threshold B, wherein a time range in which the fluctuation range of the average value ($E_M$) of the fragments is equal to or less than the predetermined threshold B is grouped as a single group among the groups.

2. The method according to claim 1,
wherein the method further comprises:
determining, using the calculator, a representative structure for the target molecule and the drug candidate molecule for each of the groups, calculating interaction energy ($E_Q$) of the representative structure determined for each of the groups in an electron state based on quantum mechanics, and calculating an expected value (E) of the interaction energy from the interaction energy ($E_Q$) calculated of the representative structure in each of the groups, and a duration of each of the groups.

3. The method according to claim 2,
wherein the expected value (E) is repeatedly calculated by fluctuating the predetermined threshold B until a fluctuation value of the expected value (E) is converged on a predetermined value.

4. A program for causing a computer to execute a method for calculating interaction energy between a target molecule and a drug candidate molecule comprising:
dividing a trajectory over a total time duration of a molecular dynamic simulation of the target molecule and the drug candidate molecule into groups based on molecular mechanic interaction energy between the target molecule and the drug candidate molecule calculated by molecular mechanics, wherein the dividing comprises:

obtaining an average value ($E_M$) of molecular mechanic interaction energy of each of fragments and the drug candidate molecules per a predetermined unit time within the total time duration, for each of the fragments over the total time duration, where the fragments are obtained by dividing the target molecule into partial structures, and the molecular mechanic interaction energy is calculated over the total time duration of the molecular dynamic simulation of the target molecule and the drug candidate molecule;

extracting the fragments having large interaction with the drug candidate molecule from all of the fragments using the average values ($E_M$) and a predetermined threshold A, wherein the fragments having the large interaction with the drug candidate molecule are identified as fragments having a representative average value ($E_{RM}$) representing all of the average values ($E_M$) that is equal to or greater than predetermined threshold A; and

dividing the trajectory over the total time duration of the molecular dynamic simulation of the target molecule and the drug candidate molecule into groups using a fluctuation range of the average value ($E_M$) of each of the fragments extracted during the molecular dynamic simulation, and a predetermined threshold B, wherein a time range in which the fluctuation range of the average value ($E_M$) of the fragments is equal to or less than the predetermined threshold B is grouped as a single group among the groups.

5. The program according to claim 4,
wherein the dividing further comprises:
determining a representative structure for the target molecule and the drug candidate molecule for each of the groups, calculating interaction energy ($E_Q$) of the representative structure determined for each of the groups in an electron state based on quantum mechanics, and calculating an expected value (E) of the interaction energy from the interaction energy ($E_Q$) calculated of the representative structure in each of the groups, and a duration of each of the groups.

**6.** The program according to claim 5,
wherein the expected value (E) is repeatedly calculated by fluctuating the predetermined threshold B until a fluctuation value of the expected value (E) is converged on a predetermined value.

**7.** A computer-readable recording medium comprising the program according to any one of claims 4 to 6 stored thereon.

**8.** A calculation device (10) configured to calculate interaction energy between a target molecule and a drug candidate molecule according to the method of any one of claim 1 to 3, the calculation device (10) comprising:
the computer-readable recording medium according to claim 7.

**Patentansprüche**

**1.** Verfahren zur Berechnung einer Wechselwirkungsenergie zwischen einem Zielmolekül und einem Wirkstoffkandidatenmolekül unter Verwendung eines Rechners, wobei das Verfahren umfasst:

Aufteilen einer Trajektorie über eine Gesamtdauer einer molekulardynamischen Simulation des Zielmoleküls und des Wirkstoffkandidatenmoleküls in Gruppen basierend auf eine durch Molekularmechanik berechnete molekularmechanische Wechselwirkungsenergie zwischen dem Zielmolekül und dem Wirkstoffkandidatenmolekül,
wobei das Aufteilen umfasst:

Erhalten, unter Verwendung des Rechners, eines Durchschnittswertes ($E_M$) der molekularmechanischen Wechselwirkungsenergie von jedem von Fragmenten und der Wirkstoffkandidatenmoleküle pro einer vorbestimmten Zeiteinheit innerhalb der Gesamtdauer, für jedes der Fragmente über die Gesamtdauer, wobei die Fragmente durch Aufteilen des Zielmoleküls in Teilstrukturen erhalten werden und die molekularmechanische Wechselwirkungsenergie über die Gesamtdauer der molekulardynamischen Simulation des Zielmoleküls und des Wirkstoffkandidatenmoleküls berechnet wird;
Extrahieren der Fragmente mit einer großen Wechselwirkung mit dem Wirkstoffkandidatenmolekül aus allen Fragmenten unter Verwendung des Rechners unter Verwendung der Durchschnittswerte ($E_M$) und eines vorbestimmten Schwellenwertes A, wobei die Fragmente mit der großen Wechselwirkung mit dem Wirkstoffkandidatenmolekül als Fragmente mit einem repräsentativen Durchschnittswert ($E_{RM}$), der alle Durchschnittswerte ($E_M$) repräsentiert, identifiziert werden, der gleich oder größer als der vorbestimmte Schwellenwert A ist; und
Aufteilen der Trajektorie über die Gesamtdauer der molekulardynamischen Simulation des Zielmoleküls und des Wirkstoffkandidatenmoleküls in Gruppen unter Verwendung des Rechners unter Verwendung einer Schwankungsbreite des Durchschnittswertes ($E_M$) jedes der während der molekulardynamischen Simulation extrahierten Fragmente, und eines vorbestimmten Schwellenwertes B, wobei ein Zeitbereich, in dem die Schwankungsbreite des Durchschnittswertes ($E_M$) der Fragmente gleich oder kleiner als der vorbestimmte Schwellenwert B ist, als eine einzelne Gruppe unter den Gruppen gruppiert wird.

**2.** Verfahren nach Anspruch 1,
wobei das Verfahren ferner umfasst:
Bestimmen, unter Verwendung des Rechners, einer repräsentativen Struktur für das Zielmolekül und das Wirkstoffkandidatenmolekül für jede der Gruppen, Berechnen der Wechselwirkungsenergie ($E_Q$) der repräsentativen Struktur, die für jede der Gruppen in einem Elektronenzustand basierend auf Quantenmechanik bestimmt wird, und Berechnen eines Erwartungswertes (E) der Wechselwirkungsenergie aus der Wechselwirkungsenergie ($E_Q$), die von der repräsentativen Struktur in jeder der Gruppen berechnet wurde, und einer Dauer jeder der Gruppen.

**3.** Verfahren nach Anspruch 2,
wobei der Erwartungswert (E) wiederholt berechnet wird, indem der vorbestimmte Schwellenwert B schwankt, bis ein Schwankungswert des Erwartungswertes (E) auf einen vorbestimmten Wert konvergiert.

**4.** Programm zur Veranlassung eines Computers ein Verfahren zur Berechnung der Wechselwirkungsenergie zwischen einem Zielmolekül und einem Wirkstoffkandidatenmolekül auszuführen, umfassend:

Aufteilen einer Trajektorie über eine Gesamtdauer einer molekulardynamischen Simulation des Zielmoleküls und des Wirkstoffkandidatenmoleküls in Gruppen basierend auf einer durch Molekularmechanik berechneten molekularmechanischen Wechselwirkungsenergie zwischen dem Zielmolekül und dem Wirkstoffkandidatenmolekül,

wobei das Aufteilen umfasst:

Erhalten eines Durchschnittswertes ($E_M$) der molekularmechanischen Wechselwirkungsenergie von jedem von Fragmenten und der Wirkstoffkandidatenmoleküle pro einer vorbestimmten Zeiteinheit innerhalb der Gesamtdauer, für jedes der Fragmente über die Gesamtdauer, wobei die Fragmente durch Aufteilen des Zielmoleküls in Teilstrukturen erhalten werden, und die molekularmechanische Wechselwirkungsenergie über die Gesamtdauer der molekulardynamischen Simulation des Zielmoleküls und des Wirkstoffkandidatenmoleküls berechnet wird;

Extrahieren der Fragmente mit großer Wechselwirkung mit dem Wirkstoffkandidatenmolekül aus allen Fragmenten unter Verwendung der Durchschnittswerte ($E_M$) und eines vorbestimmten Schwellenwertes A, wobei die Fragmente mit der großen Wechselwirkung mit dem Wirkstoffkandidatenmolekül als Fragmente mit einem repräsentativen Durchschnittswert ($E_{RM}$), der alle Durchschnittswerte ($E_M$) repräsentiert, identifiziert werden, der gleich oder größer als der vorbestimmte Schwellenwert A ist; und Aufteilen der Trajektorie über die Gesamtdauer der molekulardynamischen Simulation des Zielmoleküls und des Wirkstoffkandidatenmoleküls in Gruppen unter Verwendung einer Schwankungsbreite des Durchschnittswertes ($E_M$) jedes der während der molekulardynamischen Simulation extrahierten Fragmente, und eines vorbestimmten Schwellenwertes B, wobei ein Zeitbereich, in dem die Schwankungsbreite des Durchschnittswertes ($E_M$) der Fragmente gleich oder kleiner als der vorbestimmte Schwellenwert B ist, als eine einzelne Gruppe unter den Gruppen gruppiert wird.

**5.** Programm nach Anspruch 4,
wobei das Aufteilen ferner umfasst:
Bestimmen einer repräsentativen Struktur für das Zielmolekül und das Wirkstoffkandidatenmolekül für jede der Gruppen, Berechnen der Wechselwirkungsenergie ($E_Q$) der repräsentativen Struktur, die für jede der Gruppen in einem Elektronenzustand basierend auf Quantenmechanik bestimmt wurde, und Berechnen eines Erwartungswertes (E) der Wechselwirkungsenergie aus der Wechselwirkungsenergie ($E_Q$), die von der repräsentativen Struktur in jeder der Gruppen berechnet wurde, und eine Dauer jeder der Gruppen.

**6.** Programm nach Anspruch 5,
wobei der Erwartungswert (E) wiederholt berechnet wird, indem der vorbestimmte Schwellenwert B schwankt, bis ein Schwankungswert des Erwartungswertes (E) auf einen vorbestimmten Wert konvergiert.

**7.** Computerlesbares Aufzeichnungsmedium, das darauf gespeichert das Programm nach einem der Ansprüche 4 bis 6 umfasst.

**8.** Berechnungsvorrichtung (10), die dazu ausgelegt ist, die Wechselwirkungsenergie zwischen einem Zielmolekül und einem Wirkstoffkandidatenmolekül gemäß dem Verfahren nach einem der Ansprüche 1 bis 3 zu berechnen, wobei die Berechnungsvorrichtung (10) umfasst: das computerlesbare Aufzeichnungsmedium nach Anspruch 7.

**Revendications**

**1.** Procédé pour calculer une énergie d'interaction entre une molécule cible et une molécule candidate de médicament en utilisant un calculateur, le procédé comprenant les étapes consistant à :

diviser une trajectoire sur une durée totale d'une simulation dynamique moléculaire de la molécule cible et de la molécule candidate d'un médicament en groupes sur la base de l'énergie d'interaction mécanique moléculaire entre la molécule cible et la molécule candidate d'un médicament calculée par mécanique moléculaire, dans lequel la division comprend les étapes consistant à :

obtenir, à l'aide du calculateur, une valeur moyenne ($E_M$) d'énergie d'interaction mécanique moléculaire de chacun des fragments et des molécules candidates de médicament par unité de temps prédéterminée dans la durée totale, pour chacun des fragments sur la durée totale, où les fragments sont obtenus en divisant la molécule cible en structures partielles, et l'énergie d'interaction mécanique moléculaire est calculée sur la durée totale de la simulation dynamique moléculaire de la molécule cible et de la molécule candidate de médicament ;

extraire, à l'aide du calculateur, les fragments présentant une interaction importante avec la molécule candidate de médicament à partir de tous les fragments à l'aide des valeurs moyennes ($E_M$) et d'un seuil prédéterminé A, dans lequel les fragments présentant l'interaction importante avec la molécule candidate de médicament sont identifiés comme des fragments présentant une valeur moyenne représentative ($E_{RM}$) représentant la totalité des valeurs moyennes ($E_M$) qui est égale ou supérieure au seuil prédéterminé A ; et

diviser, à l'aide du calculateur, la trajectoire sur la durée totale de la simulation dynamique moléculaire de la molécule cible et de la molécule candidate de médicament en groupes à l'aide d'une plage de fluctuation de la valeur moyenne ($E_M$) de chacun des fragments extraits pendant la simulation dynamique moléculaire, et d'un seuil prédéterminé B, dans lequel une plage de temps dans laquelle la plage de fluctuations de la valeur moyenne ($E_M$) des fragments est égale ou inférieure au seuil prédéterminé B est groupée sous la forme d'un groupe unique parmi les groupes.

2. Procédé selon la revendication 1,
dans lequel le procédé comprend en outre l'étape consistant à :
déterminer, à l'aide du calculateur, une structure représentative pour la molécule cible et la molécule candidate de médicament pour chacun des groupes, calculer l'énergie d'interaction ($E_Q$) de la structure représentative déterminée pour chacun des groupes dans un état électronique sur la base de mécanique quantique, et calculer une valeur attendue (E) de l'énergie d'interaction à partir de l'énergie d'interaction ($E_Q$) calculée de la structure représentative dans chacun des groupes, et une durée de chacun des groupes.

3. Procédé selon la revendication 2,
dans lequel la valeur attendue (E) est calculée de manière répétée en faisant fluctuer le seuil prédéterminé B jusqu'à ce qu'une valeur de fluctuation de la valeur attendue (E) soit convergée sur une valeur prédéterminée.

4. Programme pour amener un ordinateur à exécuter un procédé pour calculer une énergie d'interaction entre une molécule cible et une molécule candidate de médicament comprenant l'étape consistant à :

diviser une trajectoire sur une durée totale d'une simulation dynamique moléculaire de la molécule cible et de la molécule candidate de médicament en groupes sur la base de l'énergie d'interaction mécanique moléculaire entre la molécule cible et la molécule candidate de médicament calculée par mécanique moléculaire,
dans lequel la division comprend les étapes consistant à :

obtenir une valeur moyenne ($E_M$) de l'énergie d'interaction mécanique moléculaire de chacun des fragments et des molécules candidates de médicament par unité de temps prédéterminée dans la durée totale, pour chacun des fragments sur la durée totale, où les fragments sont obtenus en divisant la molécule cible en structures partielles, et l'énergie d'interaction mécanique moléculaire est calculée sur la durée totale de la simulation dynamique moléculaire de la molécule cible et de la molécule candidate de médicament ;

extraire les fragments présentant une interaction importante avec la molécule candidate de médicament à partir de la totalité des fragments à l'aide des valeurs moyennes ($E_M$) et un seuil prédéterminé A, dans lequel les fragments présentant l'interaction importante avec la molécule candidate de médicament sont identifiés comme des fragments présentant une valeur moyenne représentative ($E_{RM}$) représentant la totalité des valeurs moyennes ($E_M$) qui est égale ou supérieure au seuil prédéterminé A ; et

diviser la trajectoire sur la durée totale de la simulation dynamique moléculaire de la molécule cible et de la molécule candidate de médicament en groupes à l'aide d'une plage de fluctuation de la valeur moyenne ($E_M$) de chacun des fragments extraits pendant la simulation dynamique moléculaire, et d'un seuil prédéterminé B, dans lequel une plage de temps dans laquelle la plage de fluctuation de la valeur moyenne ($E_M$) des fragments est égale ou inférieure au seuil prédéterminé B est groupée sous la forme d'un groupe unique parmi les groupes.

5. Programme selon la revendication 4,
dans lequel la division comprend en outre les étapes consistant à :
déterminer une structure représentative pour la molécule cible et la molécule candidate de médicament pour chacun des groupes, calculer l'énergie d'interaction ($E_Q$) de la structure représentative déterminée pour chacun des groupes dans un état électronique sur la base de mécanique quantique, et calculer une valeur attendue (E) de l'énergie d'interaction à partir de l'énergie d'interaction ($E_Q$) calculée de la structure représentative dans chacun des groupes, et d'une durée de chacun des groupes.

**6.** Programme selon la revendication 5,
dans lequel la valeur attendue (E) est calculée de manière répétée en faisant fluctuer le seuil prédéterminé B jusqu'à ce qu'une valeur de fluctuation de la valeur attendue (E) soit convergée sur une valeur prédéterminée.

**7.** Support d'enregistrement lisible par ordinateur comprenant le programme selon l'une quelconque des revendications 4 à 6 stocké sur celui-ci.

**8.** Dispositif de calcul (10) configuré pour calculer une énergie d'interaction entre une molécule cible et une molécule candidate de médicament selon le procédé selon l'une quelconque des revendications 1 à 3, le dispositif de calcul (10) comprenant :
le support d'enregistrement lisible par ordinateur selon la revendication 7.

## FIG. 1

## FIG. 2A

## FIG. 2B

FIG. 2C

FIG. 2D

# FIG. 3

```
                          Start

            • Fragmentation of protein
            • Setting thresholds A and B  n=0

            • MD calculation
            • Calculation of MM interaction between each fragment and ligand

            • Calculation of average value (E_M)

            • Extraction of fragments

                                      NO
                    n=0 ?                      • Threshold B = B×α (α < 1)

                    YES

            • Grouping of resultant structures of MD

            • Determination of representative structure for each group

            • Calculation of QM interaction energy (E_Q) of representative structure

            • Calculation of expected value (E)

                                   YES
                    n=0 ?

                    NO                         NO
                                                         n=n+1
            Expected value (E) is converged?

                    YES

                          End
```

# FIG. 4

# FIG. 5

## FIG. 6

## FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- The Fragment Molecular Orbital Method: Practical Applications to Large Molecular Systems. CRC Press, 2009
- **HOU T. et al.** Predictions of Binding of a Diverse Set of Ligands to Gelatinase-A by a Combination of Molecular Dynamics and Continuum Solvent Models. *JOURNAL OF PHYSICAL CHEMISTRY PART B,* 2002, vol. 106 (21), 5527-553
- **PAN D. et al.** Exploring the molecular basis of dsRNA recognition by NS1 protein of influenza A virus using molecular dynamics simulation and free energy calculation. *ANTIVIRAL RESEARCH,* 2011, vol. 92 (3), 424-433